# EUROPÄISCHE PATENTSCHRIFT

(11) **EP 3 658 590 B1**
(45) Veröffentlichungstag und Bekanntmachung des Hinweises auf die Patenterteilung: **27.03.2024**
(21) Anmeldenummer: 18750122.6
(22) Anmeldetag: 27.07.2018
(51) Int. Cl.: C08F 2/50

(54) **PHOTOINITIATOREN FÜR LICHTHÄRTENDE ZUSAMMENSETZUNGEN**
PHOTOINITIATORS FOR LIGHT-CURABLE COMPOSITIONS
PHOTOAMORCEURS POUR COMPOSITIONS PHOTODURCISSABLES

(30) Priorität: 27.07.2017 AT 3132017
(43) Veröffentlichungstag der Anmeldung: 03.06.2020
(73) Patentinhaber: Technische Universität Wien, 1040 Wien (AT)
(72) Erfinder: LISKA, Robert, 2123 Schleinbach (AT); KNAACK, Patrick, 1160 Wien (AT); GAUSS, Paul, 1180 Wien (AT); TASCHNER, Roland, 7221 Marz (AT)
(74) Vertreter: Ellmeyer, Wolfgang
(86) Internationale Anmeldenummer: PCT/EP2018/070464
(87) Internationale Veröffentlichungsnummer: WO 2019/020805

(56) Entgegenhaltungen:
- EP-A1- 0 012 548
- DE-A1- 2 808 459
- DE-A1- 2 830 953
- DE-A1-102010 018 855
- US-A- 4 038 164
- E. A. LISSI ET AL: "Polymerization photosensitized by carbonyl compounds", JOURNAL OF POLYMER SCIENCE, POLYMER CHEMISTRY EDITION., Bd. 17, Nr. 9, 1. September 1979 (1979-09-01), Seiten 2791-2803, XP055511371, US ISSN: 0360-6376, DOI: 10.1002/pol.1979.170170916

## Beschreibung

Die vorliegende Erfindung betrifft neue Photoinitiatoren für lichthärtende Zusammensetzungen.

### STAND DER TECHNIK

Auf dem Gebiet der Photopolymerisation sind zahlreiche Initiatoren und Sensitizer (Sensibilisatoren) als Auslöser der Polymerisationsreaktion bekannt, wobei die Wirkungsweise von Initiatoren vom Typ I auf der Spaltung einer intramolekularen Bindung und von solchen vom Typ II auf der Abstraktion und intermolekularem Transfer eines Wasserstoffatoms von einem Coinitiator auf das Initiatormolekül erfolgt.

Seit der Publikation der Norrish-Reaktionen (Typ I oder II) im Jahre 1932 durch Ronald George Wreyford Norrish ist bekannt, dass Carbonylverbindungen, im speziellen Ketone, durch Licht angeregt werden können, wonach sie unter Bildung reaktiver radikalischer Zwischenprodukte zerfallen. In Lissi und Encina, J. Polym. Sci., Polym. Chem. Ed. 17(9), 2791-2803 (1979), und der darin zitierten Publikaiton Lissi et al., J. Polym. Sci., Polym. Chem. Ed. 17(1), 19-26 (1979), werden Photopolymerisationsversuche mit einer Reihe von aliphatischen Ketonen, darunter unter anderem auch Ethylpyruvat, als potenzielle Initiatoren und zwei Methacrylaten als Monomere unter Bestrahlung mit einer Quecksilberdampflampe beschrieben. Es werden jedoch weder die Initiatorkonzentrationen noch die Lichtintensität angegeben, und die Umsätze wurden gezielt unter 10 % gehalten, um im ideal stationären Reaktionsverlauf zu bleiben. Unter diesen Bedingungen zeigten erwartungsgemäß alle getesteten Carbonylverbindungen, darunter sogar das Lösungsmittel Aceton, bei Bestrahlung mit geeigneten Wellenlängen Norrish-Reaktionen unter Ausbildung von Radikalen, die jeweils in gewissen, unterschiedlichen Ausmaßen die Polymerisation der Monomere initiierten. Die dabei gemessenen Polymerisationsparameter werden mit den für zwei bekannte Initiatoren, nämlich Azobis(isobutyronitril) (AIBN) bzw. Di-tert-butylperoxid (DBP), erzielten Messwerten verglichen, wobei in einem der Versuche Aceton sogar am besten von allen Ketonen abschnitt, während Ethylpyruvat jeweils nur rund ein Zehntel der Vergleichswerte für AIBN und DBP erreichte.

Aus der Verbindungsklasse der Ketone sind seit Langem Benzophenon und Acetophenon sowie diverse Derivate davon als Initiatoren bekannt, beispielsweise α-Hydroxyalkylphenone wie etwa das in DE 28 08 459 A1 offenbarte 2-Hydroxy-2-methylpropio-phenon, das davor von Ciba Geigy, nun aber von IGM Resins unter der Bezeichnung Darocur^{®} 1173 vertrieben wird und seit den 1980er-Jahren einen der am häufigsten eingesetzten Initiatoren darstellt.

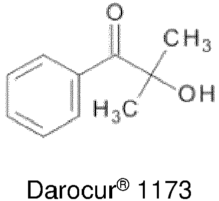

Als weitere Initiatoren auf der Basis von Acyl- und speziell Benzoyl-Gruppen werden beispielsweise in US 4.737.593 A und US 4.792.632 A Bisacyl-Derivate von Arylphosphinoxiden und in der späteren EP 615.980 A2 Bisbenzoyl-Derivate von Alkyl-, Cycloalkyl- und Arylphosphinoxiden der nachstehenden Formel offenbart: 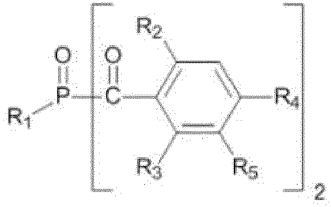 worin R₁ ein definierter Alkyl-, Cycloalkyl- oder Arylrest sein kann.

Weiters werden Glyoxylate mit aromatischen Resten seit Mitte der siebziger erfolgreich als Photoinitiatoren eingesetzt. Siehe z.B. US 4.038.164 A, worin Initiatoren der nachstehenden Formel offenbart werden: 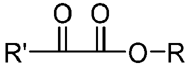 worin R unverzweiges oder verzweiges C₁₋₁₀-Alkyl, Aryl, Aralkyl oder einen Mono-, Di- oder Trialkylsilylrest darstellt und R' für einen heterozyklischen Rest, C₆₋₁₄-Aryl oder gegebenenfalls ein- oder mehrfach substituiertes Phenyl steht.

Kürzlich wurde aus WO 2017/060459 A1 und WO 2017/060527 A1 der Dentsply Detrey GmbH eine neue Gruppe von Photoinititoren bekannt, die dort "Verbindungen mit einer Acylsilyl- oder Acylgermyl-Gruppe" genannt werden, tatsächlich aber zusammenfassend eher als als Silyl- bzw. Germyl-substituierte α-Diketone bzw. α-Ketoester und -amide zu bezeichnen sind und durch die folgende Formel dargestellt werden können: 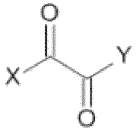 worin X für R₁R₂R₃Si- oder R₁R₂R₃Ge- steht, worin R₁ bis R₃ jeweils unabhängig einen gegebenenfalls substituierten Kohlenwasserstoffrest darstellen, so dass X für eine Trihydrocarbylsilyl- oder -germyl-Gruppe steht; und
Y entweder für dieselben Optionen wie X, wobei sich die Hydrocarbylreste von jenen in X unterscheiden können, oder aber für -Z-R₄ steht, worin Z für eine chemische Bindung, -O- oder -NR'-, worin R' ein gegebenenfalls substituierter Hydrocarbylrest ist, steht und R₄ für einen gegebenenfalls substituierten Hydrocarbylrest oder eine Trihydrocarbylsilyl-Gruppe steht, wobei einer oder zwei der Hydrocarbylreste am Silicium auch Hydrocarbylcarbonylreste sein können.

Wesentlich ist in jedem Fall das mit drei (gegebenenfalls weiter substituierten) Kohlenwasserstoffresten substituierte Silicium- oder Germaniumatom in α-Stellung zur Ketogruppe des α-Diketons bzw. α-Ketoesters oder -amids. Als bevorzugte Ausführungsformen der Silicium und/oder Germanium enthaltenden Initiatoren werden mehrheitlich diverse Derivate von Acetophenon genannt, in denen das α-Kohlenstoffatom durch Si oder Ge ersetzt wurde.

Derartige Photoinitiatoren sollen unter anderem besonders gut für die Härtung von Dentalmaterial im Mund eines Patienten geeignet sein, wobei sie eine hohe Quantenausbeute der Lichtabsorption aufweisen und nicht aus der ungehärteten Masse heraus migrieren sollen. Darüber hinaus werden auch Säurebeständigkeit, gute Löslichkeit in der polymerisierbaren Zusammensetzung, Thermostabilität und Lagerstabilität gefordert.

Diese Initiatoren werden gemäß WO 2017/060459 A1 vorzugsweise bzw. gemäß WO 2017/060527 A1 sogar zwingend in Kombination mit einem Coinitiator eingesetzt, der wofür im ersterem Fall Elektronendonatoren wie etwa Amine und in zweiterem Fall spezielle Acrylate offenbart werden. Als dadurch erzielte Vorteile werden hohe Polymerisationseffizienz und Härtungsgeschwindigkeit sowie keine Verfärbung während der Härtung genannt.

Nachteilig ist an diesen Initiatoren allerdings, dass sie allesamt gegenüber sichtbarem Licht empflindlich sind. Auch sind die Photoinitiatoren mit ihren aromatischen Resten im Bereich der Medizin und auf dem Lebensmittelsektor nur bedingt einsetzbar.

Vor diesem Hintergrund war das Ziel der vorliegenden Erfindung die Bereitstellung alternativer Photoinitiatoren auf der Basis von α-Ketoestern, mit denen die obigen Probleme zumindest teilweise gelöst werden können.

### OFFENBARUNG DER ERFINDUNG

Dieses Ziel erreicht die vorliegende Erfindung durch Bereitstellung der neuen Verwendung von Verbindungen - genauer gesagt α-Ketoestern - der nachstehenden Formel (I) als Photoinitiator oder Photosensitizer in einer photopolymerisierbaren Zusammensetzung: 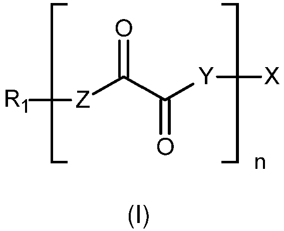 worin R₁ für einen einwertigen, linearen, verzweigten oder zyklischen aliphatischen Kohlenwasserstoffrest mit 1 bis 20 Kohlenstoffatomen steht, der gegebenenfalls durch ein oder mehrere Heteroatome, ausgewählt aus -O- und -NR₂- unterbrochen ist und der gegebenenfalls mit einem oder mehreren Substituenten, ausgewählt aus -Cl, -Br, -OH, =O, -NH-CO-OR₂, -NH-CO-R₂ und radikalisch oder ionisch polymerisierbaren Gruppierungen substituiert ist, wobei die Reste R₂ jeweils unabhängig aus -H und C₁₋₆-Alkyl ausgewählt sind;
n ≥ 1 ist, wobei
i) wenn n = 1 ist, Z und Y fehlen und X für -OR₃ steht; und
ii) wenn n > 1 ist, Z für -OR₄- steht, Y für -OR₅- steht und X für -H oder -OH steht; worin
   R₃ für -H oder eine der Optionen für R₁ steht; und
   R₄ und R₅ jeweils unabhängig für einen zweiwertigen Kohlenwasserstoffrest stehen, für den ansonsten dieselben Optionen wie für R₁ gelten;

wobei die polymerisierbaren Gruppierungen als optionale Substituenten von R₁ aus polymerisierbaren Doppel- und Dreifachbindungen sowie aus der Ringöffnungspolymerisation zugänglichen Lactam-, Lacton- und Epoxid-Gruppierungen ausgewählt sind;
und wobei beliebige zwei der Reste R₁ bis R₅ gegebenenfalls miteinander verbunden sind, um einen Ring oder ein Dimer zu bilden;
mit der Maßgabe, dass die Verbindung der Formel (I) nicht 2-Oxopropansäureethylester (Brenztraubensäureethylester, Ethylpyruvat) (1) ist.

Bei der erfindungsgemäßen Verwendung der obigen Verbindungen der Formel (I) als Photoinitiatoren sind diese in der Regel zumindest gleichwertig zu bekannten Initiatoren, und zwar sowohl zu Standardinitiatoren wie etwa Benzophenon als auch zu den relativ neuen Verbindungen gemäß WO 2017/060459 A1 bzw. WO 2017/060527 A1, in vielerlei Hinsicht übertreffen sie diese jedoch sogar. Aufgrund des Fehlens aromatischer Reste und benachbarter Si- oder Ge-Atome sind die erfindungsgemäß eingesetzten α-Ketoester zudem physiologisch unbedenklich und neigen auch unter dem Einfluss von sichtbarem Licht nicht zur Zersetzung. Die Verbindungen der Formel (I) sind daher bestens für die Verwendung als Photoinitiatoren bzw. -sensitizer geeignet, wie die späteren Beispiele klar belegen.

In bevorzugten Ausführungsformen der vorliegenden Erfindung steht R₁ für einen einen linearen, verzweigten oder zyklischen aliphatischen Kohlenwasserstoffrest mit 1 bis 20 Kohlenstoffatomen, in dem gegebenenfalls ein oder mehrere Kohlenstoffatome durch Sauerstoffatome ersetzt sind und der gegebenenfalls mit einem oder mehreren Substituenten, ausgewählt aus -Cl, -Br, -OH und -SH substituiert ist, und/oder ist n = 1 und steht X für -OR₃, wobei R₃ jeweils unabhängig für -H oder eine der Optionen für R₁ steht.

Unter einem Dimer einer Verbindung der Formel (I) ist eine Verbindung zu verstehen, die durch formale Verknüpfung eines beliebigen der Reste R₁ bis R₄ einer Verbindung der Formel (I) mit einem beliebigen der Reste R₁ bis R₄ derselben oder einer anderen Verbindung der Formel (I) gebildet wird. Für Verbindungen der Formel (I) mit n = 1 und X = -OR₃ ergeben sich im Falle von endständiger Verknüpfung beispielsweise jeweils drei Optionen, nämlich über die beiden Reste R₁, über jeweils einen Rest R₃ der beiden Gruppierungen X oder über jeweils einen Rest R₁ und einen Rest R₃ einer Gruppierung X, wie dies nachstehend dargestellt ist:

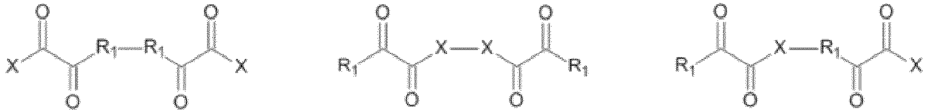

In im Wesentlichen analoger Weise können zwei Moleküle der Formel (I), in denen zumindest einer der Reste R₁ und R₃ (wenn n = 1 ist) bzw. R₄ (wenn n > 1 ist) einen C₁₋₆-Alkylrest als Rest R₂ umfasst, d.h. -NR₂- als Ersatz für ein Kohlenstoffatom bzw. einen Substituenten -NH-CO-OR₂ oder -NH-CO-R₂ umfasst, über diese Reste R₂ verbunden sein - sei es, dass zwei Reste R₂ miteinander oder jeweils ein Rest R₂ mit einem Rest R₁, R₃ oder R₄ des anderen Moleküls verbunden ist. Verknüpfungen zwischen jeweils einem Rest R₁ und einem Rest R₃ bzw. R₄ oder zwischen zwei Resten R₃ bzw R₄ sind allerdings ebenfalls umfasst.

Ähnliches gilt für einen optionalen intramolekularen Ringschluss zwischen zwei beliebigen der Reste R₁ bis R₃ (wenn n = 1 ist) bzw. R₄ (wenn n > 1 ist) desselben Moleküls. Nachstehend ist exemplarisch wiederum eine endständige Verknüpfung für den Fall n = 1 zwischen R₁ und dem Rest R₃ (oder einem davon umfassten Rest R₂) der Gruppierung X über einen dazwischen liegenden Pentylenrest dargestellt, der somit als Teil entweder des Rests R₁ oder des Rests R₃ (oder R₂) der Gruppierung X angesehen werden kann:

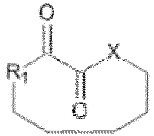

Neben den oben erwähnten bevorzugten Ausführungsformen der erfindungsgemäßen α-Ketoester, in denen n = 1 ist, Z und Y fehlen und X für -OR₃ steht, so dass R₁ und X jeweils direkt an eine der beiden Gruppierungen C=O gebunden sind, sind jedoch auch spezielle Ausführungsformen bevorzugt, in denen n > 1 ist, Z für -OR₄- steht, Y für -OR₅- steht und X für -H oder -OH steht. Dabei handelt es sich somit um Oligo- oder Polymere von Glyoxylsäureestern, in denen das photoaktive Glyoxylsäure-Grundmotiv n-mal enthalten ist. Besonders bevorzugt sind Ausführungsformen, in denen in der Gruppierung Z das zum Sauerstoffatom benachbarte Kohlenstoffatom des Rests R₄ mit einer Oxo-Gruppe =O substituiert ist, woraus eine Caboxylgruppe resultiert, und in denen X für -OH steht. Solche Moleküle sind folglich Polyester, die durch Polykondensation von α-Ketodicarbonsäuren mit Diolen vergleichsweise einfach zugänglich sind, weswegen Ausführungsformen besonders bevorzugt sind, in denen R₁ eine endständige OH-Gruppe aufweist, wie dies nachstehend allgemein dargestellt ist:

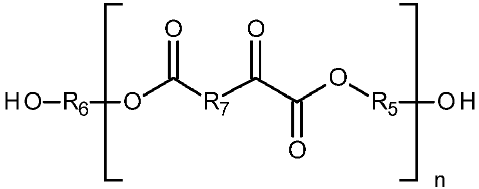

Dabei stehen HO-R₆- für den Rest R₁- und -(C=O)-R₇- für den Rest -R₄- in Formel (I), und X ist -OH. Somit definiert der Rest R₄ bzw. R₇ die Dicarbonsäure und der Rest R₅ das Diol, aus denen solche Polymere erhältlich sind. Beispielsweise ergibt -(CH₂)₂- als Rest R₇ α-Ketoglutarsäure und -(CH₂)₆- als Reste R₅ und R₆ Hexandiol als Ausgangsprodukte der Polykondensation, wie dies in den späteren Beispielen offenbart wird.

Der Wert für n definiert die Kettenlänge der Verbindungen der Formel (I) und ist prinzipiell nicht speziell eingeschränkt, solange die Produkte auf die erfindungsgemäße Weise als Photoinitiator oder Photosensitizer wirken. Da bei Bestrahlung solcher Polymere der Formel (I) gleichzeitig eine Vielzahl von Radikalzentren gebildet wird, sorgen solche Photoinitiatoren oder -sensitizer für besonders rasche Polymerisationsreaktionen. Aufgrund einfacherer Handhabbarkeit wird gemäß vorliegender Erfindung als Obergrenze für n ein Wert von 100 bevorzugt, und ein Wert von 50 ist noch bevorzugter. Mitunter sind jedoch auch deutlich niedrigere Werte, beispielsweise im Bereich von 2 bis 10, zu bevorzugen.

Es versteht sich für den Fachmann, dass die Verbindungen der Formel (I) nicht nur miteinander zu Dimeren verbunden, sondern auch an andere Moleküle gebunden sein können. Speziell OH-Endgruppen, wie sie in den oben beschriebenen bevorzugten Ausführungsformen der Erfindung unter Verwendung von Polyestern der Formel (I) enthalten sind, sind Standard-Derivatisierungsreaktionen leicht zugänglich und können auf einfache Weise beispielsweise an Präpolymere und andere Polymerbausteine in den Formulierungen, in denen sie als Initiatoren oder Sensitizer zum Einsatz kommen sollen, gebunden oder auch an feste Phasen immobilisiert werden. Auch die Bildung von Alkoholaten, vorzugsweise mit Alkalimetall-Kationen wie Li⁺, Na⁺ oder K⁺, kann unter bestimmten Umständen vorteilhaft sein. Außerdem versteht es sich, dass manche der in den Verbindungen der Formel (I) enthaltenen Sauerstoffatome durch Schwefelatome ersetzt sein können, sofern die Reaktivität der Moleküle als Photoinitiatoren oder Sensitizer dadurch nicht verloren geht.

Die Verbindung der Formel (I) kann gemäß vorliegender Erfindung sowohl als Photoinitiator vom Typ I als auch vom Typ II eingesetzt werden, d.h. je nach den Reaktionsbedingungen kann entweder eine intramolekulare Bindung gespalten oder ein Wasserstoffatom von einem Lösungsmittel- oder Coinitiator-Molekül abstrahiert und auf den Initiator der Formel (I) übertragen werden, wie dies die späteren Ausführungsbeispiele belegen.

Daher wird die Verbindung der Formel (I) in bevorzugten Ausführungsformen der vorliegenden Erfindung, speziell in solchen, in denen sie als Photoinitiator vom Typ II dient, in Kombination mit einem oder mehreren Coinitiatoren in den photopolymerisierbaren Zusammensetzungen eingesetzt.

Als Coinitiator werden dabei vorzugsweise eine oder mehrere Verbindungen eingesetzt, die aus ein- oder mehrwertigen Alkoholen (-OH), Thiolen (-SH), Aminen (-NR-), Silanen (=SiH-), Germanen (=GeH-), Phosphinen (-PRR'R"-), Ethern (>CH-O-CH<), lodonium- (-I⁺-) und Sulfonium- (=S⁺-) Salzen sowie Derivaten davon ausgewählt sind, da sich derartige Verbindungen in der Vergangenheit als Coinitiatoren bewährt haben.

Noch bevorzugter werden als Coinitiator eine oder mehrere Verbindungen, ausgewählt aus Polyolen auf Basis von Zuckern, Glycerin oder dergleichen, Thiolen, Polyethylenglykol und Polypropylenglykol eingesetzt, wobei als mehrwertiger Alkohol insbesondere Glycerin eingesetzt wird, das sich in den Ausführungsbeispielen als Coinitiator für die Verbindungen der Formel (I) besonders bewährt hat.

Die Verbindung der Formel (I) wird dabei vorzugsweise in einer Menge von 0,1 bis 10 Gewichtsteilen, noch bevorzugter 0,5 bis 5 Gewichtsteilen, insbesondere etwa 1 bis 2 Gewichtsteilen, pro 100 Gewichtsteile an polymerisierbaren Monomeren in der Zusammensetzung eingesetzt.

Die Verbindung der Formel (I) wird zudem vorzugsweise zur Härtung von radikalisch polymerisierbaren Monomeren wie Acrylaten, Methacrylaten, Maleinimiden, Styrolderivaten, Vinylestern, Vinylcarbonaten, Vinylcarbamaten und dergleichen sowie ringöffnenden Monomeren, wie z.B. Vinylcyclopropanen, Vinylcyclooxiranen, Vinylcyclobutanen, Ketenacetalen, Vinylspiroestern und dergleichen, noch bevorzugter zur Härtung von Acrylat-, Methacrylat-, Styrol- oder Maleinimid-Monomeren, besonders bevorzugt zur Härtung von Acrylat- und Methacrylat-Monomeren, eingesetzt, da sie sich in solchen Zusammensetzungen gegenüber Standardinitiatoren deutlich überlegen erwiesen haben.

Aufgrund ihrer physiologischen Unbedenklichkeit können die Verbindungen der Formel (I) darüber hinaus bevorzugt als Initiatoren in Zusammensetzungen eingesetzt werden, die innerhalb des menschlichen Körpers gehärtet werden sollen, oder auch für härtbare Zusammensetzungen auf dem Lebensmittelsektor. Dies zeichnet sie gegenüber der Mehrzahl der Standardinitiatoren aus.

Weitere Komponenten der polymerisierbaren Zusammensetzungen sind nicht speziell eingeschränkt, solange sie keine nachteiligen Auswirkungen auf die Wirksamkeit der Initiatoren und den Härtungsvorgang haben. Folglich können beispielsweise beliebige geeignete Füllstoffe, Lösungsmittel, weitere Initiatoren oder Sensitizer, Weichmacher, Fließhilfen und dergleichen eingesetzt werden.

In einem zweiten Aspekt betrifft die vorliegende Erfindung daher eine photopolymerisierbare Zusammensetzung, die dadurch gekennzeichnet ist, dass sie zumindest eine Verbindung der Formel (I), die nicht 2-Oxopropansäureethylester (1) ist, als Photoinitiator oder Photosensitizer, zumindest ein photopolymerisierbares Monomer und gegebenenfalls zumindest einen Coinitiator, sowie gegebenenfalls weitere Komponenten, wie zuvor definiert, umfasst.

### BEISPIELE

Die vorliegende Erfindung wird nachstehend anhand von repräsentativen Beispielen näher beschrieben, die ausschließlich zur Illustration der Erfindung dienen, nicht aber als Einschränkung zu verstehen sind.

### Materialien und Verfahren

Alle Reagenzien und Photoinitiatoren wurden, sofern nachstehend nichts anderes angegeben ist, aus kommerziellen Quellen bezogen und ohne weitere Reinigung eingesetzt. Die ¹H- und ¹³C-NMR-Spektren wurden großteils auf einem Bruker DPX-200 Fourier Transform-Spektrometer bei 200 MHz bzw. 50 MHz aufgenommen, und manche Messungen auf einem Bruker Avance bei 400 MHz (¹H) bzw. 100 MHz (¹³C). Die Massenspektren wurden auf einem Thermo Fisher Scientific ITQ 1100 unter Verwendung einer Kieselgel-Kapillarsäule (30 m x 0,25 mm) aufgenommen.

In den Beispielen für die vorliegende Erfindung wurden die folgenden Photoinitiatoren (2) bis (13) der Formel (I) sowie die nicht erfindungsgemäße Verwendung des aus dem Stand der Technik bekannten Ethylpyruvats (1) getestet:
2-Oxopropansäureethylester (Brenztraubensäureethylester, Ethylpyruvat) (1) (kein Beispiel der vorliegenden Erfindung): 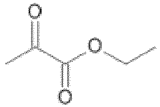
2-Oxobutansäuremethylester (2-Oxobuttersäuremethylester) (2): 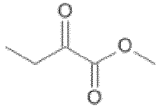
2-Oxo-3-methylbutansäureethylester (2-Oxoisovaleriansäureethylester) (3): 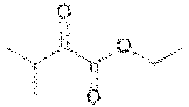
2-Oxo-3,3-dimethylbutansäuremethylester (4): 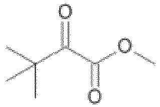
3-Brom-3-methyl-2-oxobutansäureethylester (5): 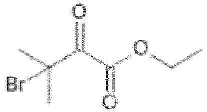
3-Hydroxy-3-methyl-2-oxobutansäureethylester (6): 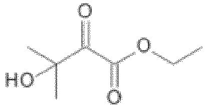
2-Oxopropansäure-2-(N,N'-dimethylamino)ethylester (2-(N,N'-Dimethylamino)ethylpyruvat) (7) 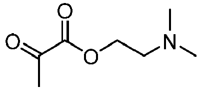
4,4-Dimethyldihydrofuran-2,3-dion (8): 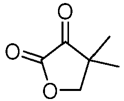
α-Ketoglutarsäurediethylester (9): 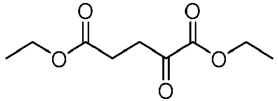
3,3-Dimethyl-2-oxobutansäure-2-(N,N'-dimethylamino)ethylester (10) 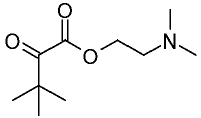
α-Ketoglutarsäuredi(hydroxyethylmethacrylat)ester (11): 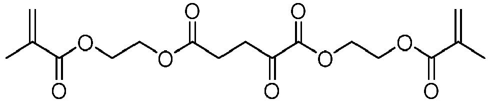
α-Ketoglutarsäurehexandiol-Polyester (Mn -10000 Da) (12) 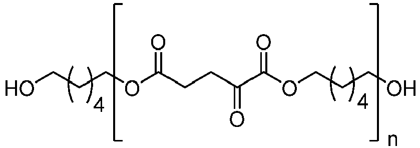
Urethanmetharylat-terminierter α-Ketoglutarsäurehexandiol-Polyester (13)

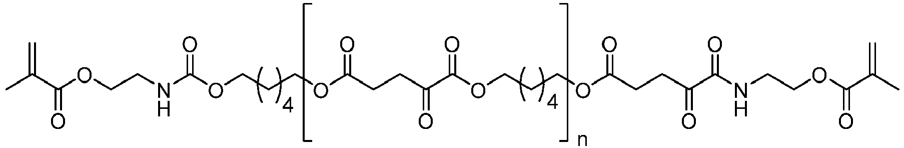

Dabei wurden die Initiatoren (1) bis (4) aus kommerziellen Quellen bezogen und die neuen Initiatoren (5) bis (13) von den Erfindern selbst synthetisiert, wie in den späteren Synthesebeispielen ausführlich beschrieben wird.

In den Vergleichsbeispielen wurden vier bekannte, kommerziell erhältliche Initiatoren getestet, nämlich:
Benzophenon (BP): 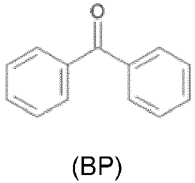
Ethylphenylglyoxylat (PGO) 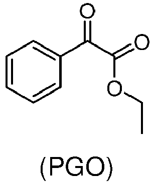
2-Brom-2-methyl-1-phenyl-propan-1-on (2-Bromisobutyrophenon) ("Bromdarocur", BD): 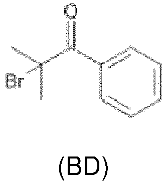
3-(4-Benzoylphenoxy)-2-hydroxy-N,N-dimethylpropan-1-aminhydrochlorid (BPQ)

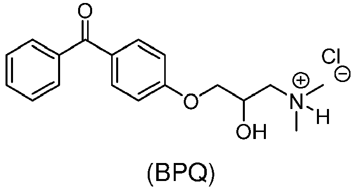

### Synthesebeispiel 1

### Herstellung von 3-Brom-3-methyl-2-oxobutansäureethylester (5)

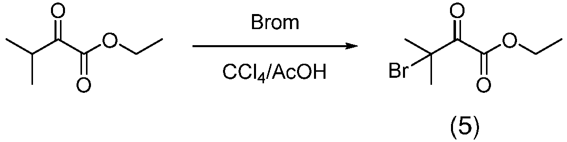

In einem 50-ml-Dreihalskolben wurden 15 ml CCl₄ vorgelegt. Dazu wurde 1 Äquivalent (5,62 g, 39 mmol) 3-Methyl-2-oxobutansäureethylester zugesetzt und danach 1 Äqu. (6,24 g, 39 mmol) Brom zugegeben. Anschließend wurde mit 1,5 ml Essigsäure angesäuert. Nach dem Entfärben der Lösung wurde die Reaktion mit etwa 30 ml ges. NaHCO₃-Lösung gequencht und das Gemisch in einen Scheidetrichter übergeführt. Dort wurden 100 ml Diethylether und weitere 20 ml NaHCO₃ zugesetzt. Die wässrige Phase wurde verworfen und die organische Phase erneut mit 50 ml NaHCO₃ extrahiert. Die Etherphase wurde mit ges. NaCl-Lösung gewaschen und über Natriumsulfat getrocknet. Anschließend wurden die Lösungsmittel am Rotationsverdampfer abgezogen.
Ausbeute: 6,94 g (79 % d. Th.)
¹H-NMR (400 MHz, C₆D₆) δ ppm: 4,31 (q, 2 H, J=7,02 Hz); 1,95 (s, 6 H); 1,32 (t, 3 H, J=7,2 Hz).
GC-MS: 224,99 [M+H]⁺, 143,07 [M-Br]⁺, 122,93 [M-COCOOEt]⁺, 70,20 [M-Br-COOEt]⁺.

### Synthesebeispiel 2

### Herstellung von 3-Hydroxy-3-methyl-2-oxobutansäureethylester (6)

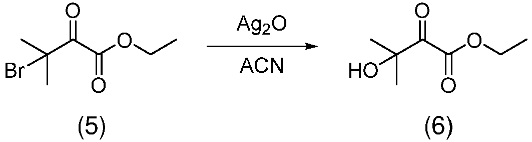

1 Äquivalent (4,46 g, 20 mmol) 3-Brom-3-methyl-2-oxobutansäureethylester wurde zu 0,5 Äqu. Silber(I)-oxid in 40 ml feuchtem Acetonitril (ACN) hinzugefügt. Das Gemisch wurde 12 h lang gerührt und der gebildete hellgraue Niederschlag abfiltriert. Danach wurde die Lösung mit 100 ml Wasser verdünnt und mit 200 ml Ether extrahiert und die organische Phase über Natriumsulfat gerocknet. Nach dem Abziehen des Lösungsmittels wurde das Rohprodukt fraktioniert destilliert (120 °C, 25 mbar).
Ausbeute: 1,50 g (93 % d. Th.)
¹H-NMR (400 MHz, C₆D₆) δ ppm: 3,85 (q, 2H, J=7,31 Hz); 2,94 (bs, 1 H); 1,30 (s, 6H); 0,83 (t, 3H, J= 7,09 Hz).
GC-MS: 161,16 [M+H]⁺, 143,20 [M-OH]⁺, 115,16 [M-OEt]⁺, 87,09 [M-COOEt]⁺, 59,06 [(CH₃)₂OH]⁺.

### Synthesebeispiel 3

### Herstellung von 3,3-Dimethyl-2-oxobutansäure-2-(N,N'-dimethylamino)ethylester (10)

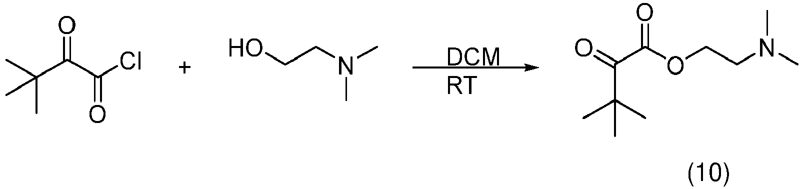

In einem 100-ml-Dreihalsrundkolben wurden unter starken Rühren und gleichzeitiger Eisbadkühlung zu einem Gemisch aus 1 Äq. 3,3-Dimethyl-2-oxobutanoylchlorid in 10 ml Dichlormethan 2,5 Äq. N,N-Dimethylethanolamin über ein Septum zugetropft. Nach vollendeter Zugabe wurde 48 h lang bei Raumtemperatur weitergerührt. Nach beendeter Reaktion wurde das Lösungsmittel mittels Rotatinsverdampfer abgezogen. Das Produkt wurde mittels Kugelrohrdestillation bei 1,2 mbar und 94 °C als klares Öl gewonnen.
Ausbeute: 177 mg (17 % d.Th.)
¹H-NMR (400 MHz, C₆D₆, ppm): 4,11 (t, 2 H), 2,29 (t, 2H), 2,02 (s, 6 H), 1,16 (s, 9 H).

Mischung von 1 Äq. 3,3-dimethyl-2-oxobutanoylchlorid in 10 ml Dichlormethan 2,5 Äq. N,N-Dimethylethanolamin über ein Septum zugetropft. Nach vollendeter Zugabe wurde für 48 Stunden bei Raumtemperatur weitergerührt. Nach beendeter Reaktion wurde das Lösungsmittel mittels Rotatinsverdampfer abgezogen Das Produkt wurde mittels Kugelrohrdestillation bei 1,2 mbar und 94 °C als klares Öl gewonnen. Ausbeute 177 mg (17 % d. Th.)

¹H-NMR (400 MHz, C₆D₆, ppm): 4,11 (t, 2 H), 2,29 (t, 2H), 2,02 (s, 6 H), 1,16 (s, 9 H)

### Synthesebeispiel 4

### Herstellung von α-Ketoglutarsäurediethylester (9)

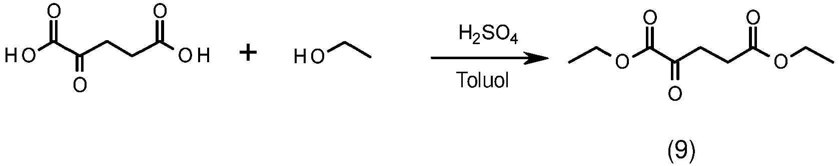

Zunächst wurde die α-Ketoglutarsäure aus Aceton umkristallisiert. Dann wurde 1 Äq. (3,662 g, 25 mmol) α-Ketoglutarsäure in einen 250-ml-Dreihalsrundkolben, ausgestattet mit Kühler und Septum, eingewogen, wonach 100 ml Ethanol und 0,3 Äq. (0,4 ml, 7,5 mmol) Schwefelsäure zugesetzt wurden. Das Reaktionsgemisch wurde magnetisch gerührt und rückflusserhitzt. Das Ölbad wurde dabei auf 100 °C eingestellt und die Reaktion 24 h lang laufen gelassen. Der Reaktionsfortschritt wurde mittels Dünnschichtchromatographie überprüft (PE:EE = 5:1; Rf: 0,36). Das Lösungsmittel wurde am Rotationsverdampfer angedampft, wonach 50 ml deionisiertes Wasser zugesetzt wurden unf die Lösung mit 1 N KOH-Lösung neutralisiert wurde. Die wässrige Phase wurde dreimal mit 100 ml Ethylacetat extrahiert und die vereinigten organischen Phasen mit Na₂SO₄ getrocknet. Dann wurde das Lösungsmittel am Rotationsverdampfer angedampft. 4,193 g (83 % d. Th.) Rohausbeute wurde erhalten. Das Rohprodukt wurde mittels MPLC auf 214 g Kieselgelsäule (PE:EE = 9:1) chromatographisch getrennt. Das Produkt wurde über einen UV-Detektor (254 nm) detektiert und das Produkt mittels DC identifiziert (PE:EE = 5:1; Rf: 0,36). Die Fraktionen, die das Produkt enthielten, wurden vereinigt und das Lösemittel am Rotationsverdampfer abgedampft. Insgesamt wurden 1,77 g (35 % d. Th.) des α-Ketoglutarsäurediethylesters (9) als farbloses Öl erhalten.
Rf: 0,36 (PE:EE = 5:1)
¹H-NMR (400 MHz, CDCl₃, ppm): 4,32 (q, J=7,2 Hz, 2H), 4,13 (q, J=7,20 Hz, 2H), 3,14 (t, J=6,6 Hz, 2H), 2,65 (t, J=6,6 Hz, 2H), 1,36 (t, J=7 Hz, 3H), 1,24 (t, J=7 Hz, 3H).
¹³C-NMR (400 MHz, CDCl₃, ppm): 192,8, 172,0, 160,6, 62,6, 60,9, 34,2, 27,8, 14,2, 14,0.
GC-MS: 202,99 [M+H], 129,94 [M+H,-(CO-O-CH₂-CH₃)], 128,95 [M-(CO-O-CH₂-CH₃)], 101,99 [M+H,-(CO-CO-O-CH₂-CH₃)], 101,00 [M-CO-CO-O-CH₂-CH₃], 74,11 [M+H, -(CO-CH₂-CH₂-CO-O-CH₂-CH₃)], 73,14 [M-(CO-CH₂-CH₂-CO-O-CH₂-CH₃)], 55,13 [M+H,-(O-CH₂-CH₃),-(CH₂-CH₂-CO-O-CH₂-CH₃)].

### Synthesebeispiel 5

### Herstellung von α-Ketoglutarsäure-di-2-hydroxyethylmethacrylsäureester (11)

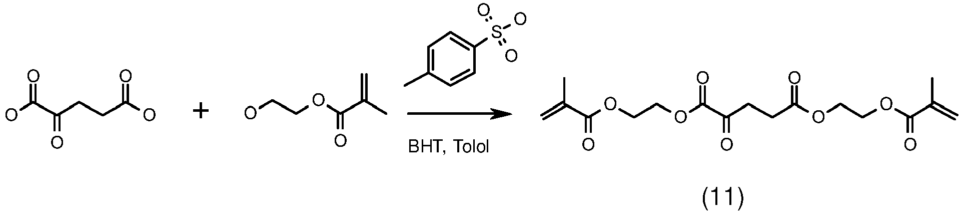

Zunächst wurde die α-Ketoglutarsäure aus Aceton umkristallisiert. Dann wurden 1 Äq. (3,943 g, 27 mmol) α-Ketoglutarsäure, 2 Äq. (7,290 g, 54 mmol) 2-Hydroxymethylmethacrylat, 0,7 Gew.-% (78,5 mg) Lipase (Candida Antarctica) immobilisiert auf Acrylharz (<5,000 U/g) und 565 ppm (10,1 mg) of 2,6-Di-tert-butyl-4-methylphenol (BHT) in einem 50 ml Einhalsrundkolben vorgelegt. Dieser wurde mit einem Trockenrohr, gefüllt mit Calciumchlorid, ausgestattet. Das Gemisch wurde in einem Ölbad mit 65 °C magnetisch gerührt. Der Reaktionsfortschritt wurde mittels NMR und DC (PE:EE = 8:2; Rf: 0,62) überprüft, und nach einer Reaktionszeit von 140 h wurde das Reaktionsgemisch mit PE:EE (1:1) verdünnt und auf einer 90-g-Kieselgelsäule mittels MPLC (PE:EE = 8:2) chromatographisch getrennt. Dann wurden die vereinigten Produktfraktionen mit BHT versetzt und am Rotationsverdampfer das Lösungsmittel abgedampft. Es wurden 2,991 g (30 % d. Th.) des reinen Produkts als farbloses, transparentes Öl erhalten.
Rf: 0,62 (PE:EE = 8:2)
¹H-NMR (400 MHz, (Aceton-d₆, ppm): 6,05 (s, 2H, 2 CHH), 5,62-5,59 (m, 2H), 4,54-4,48 (m, 2H), 4,43-4,38 (m, 2H), 4,30 (s, 4H), 3,15 (t, J=6,6 Hz, 2H), 2,63 (t, J=6,6 Hz, 2H), 1,87 (t, J=1,2 Hz, 6H).

### Synthesebeispiel 6

### Herstellung von Ketoglutarsäurehexandiol-Polyester (12)

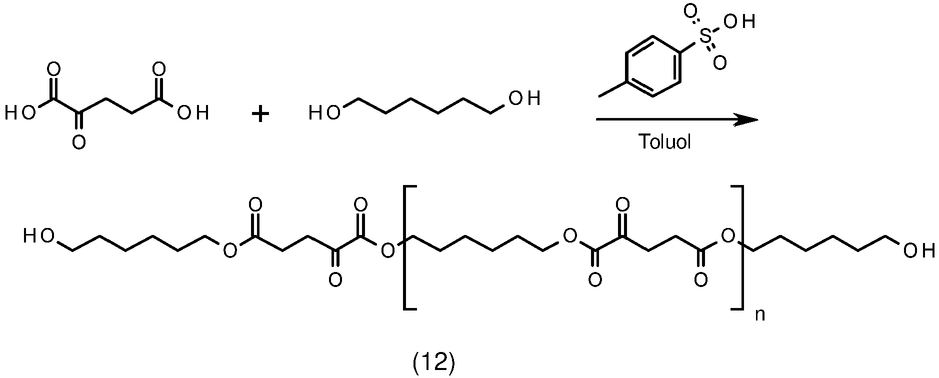

Zunächst wurden die α-Ketoglutarsäure aus Aceton und die p-Toluolsulfonsäure aus Chloroform umkristallisiert. Dann wurden 1 Äq. (11,18 g, 80 mmol) der reinen α-Ketoglutarsäure, 1,01 Äq. (9,54 g, 81 mmol) 1,6-Hexandiol und 0,0025 Äq. (30,2 mg, 0,2 mmol) der reinen p-Toluolsulfonsäure in einen 100 ml Dreinhalskolben mit magnetischer Rührung und einem Dean-Stark Wasserabscheider eingewogen. 30 ml abs. Toluol wurden hinzugefügt, und das Ölbad wurde auf 125 °C erhitzt. Der Reaktionsfortschritt wurde mittels NMR überwacht und nach 24 h die Reaktion abgebrochen. Der Polyester in Lösung wurde mit 30 ml abs. Toluol verdünnt und in 800 ml kaltem Diethylether ausgefällt. Das Resultat war ein leicht gelblicher Polyester, der im Vakuumtrockenschrank bei 50 °C getrocknet wurde, woraufhin er in 60 ml THF gelöst, die Lösung filtriert und erneute Fällung in 800 ml kaltem Diethylether folgten. Nach dem Trocknen im Vakuum wurden 11,36 g (62%) des Polyesters als weißes Polymer erhalten.
Molekulargewicht Mn (mittels GPC und NMR) ≈ 10.000; n ≈ 40
¹H-NMR (400 MHz, CDCl₃, ppm): 4,26 (t, J_{HH}=6,6 Hz, 40 H), 4,08 (t, J_{HH}=6,6 Hz, 40 H), 3,65 (t, J_{HH}=6,6 Hz, 4H), 3,15 (t, J_{HH}=6,6 Hz, 40H), 2,67 (t, J_{HH}=6,6 Hz, 40H), 1,78-1,72 (m, 40H), 1,68-1,60 (m, 40H), 1,45-1,36 (m, 80H).

### Synthesebeispiel 7

### Herstellung von α-Ketoglutarsäure-1,6-Hexandiol-Polyester mit 2-Isocyanatoethylmethacylat-modifizierten Endgruppen (13)

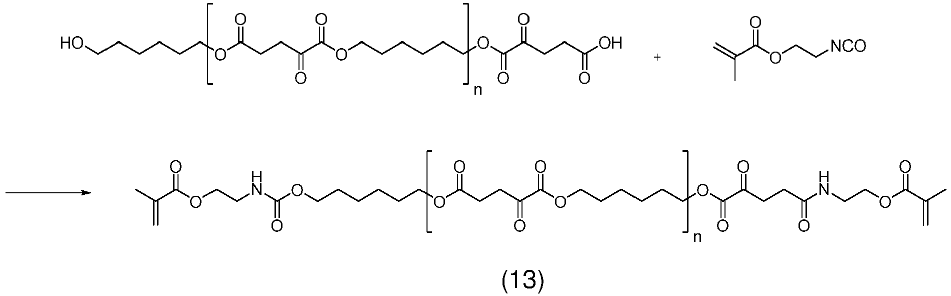

Im ersten Schritt wurden 1 Äq. des Polyesters (0,98 g, 0,2 mmol), 2 Tropfen Dibutylzinndilaurat als Katalysator und 20 ml abs. Toluol in einem 50-ml-Dreihalsrundkolben vorgelegt. Der Kolben wurde mit Argon gespült und mit einem Septum und einem Argon-Ballon ausgestattet. Dann wurden über das Septum 2,05 Äq. (0,6 ml, 0,4 mmol) 2-Isocyanatoethylmethacylat zugetropft. Das Gemisch wurde 14 h lang bei Raumtemperatur gerührt und anschließend mit 5 ml Methanol gequencht. Es wurden 20 ml destilliertes Aceton zugegeben und der Polyester in 300 ml kaltem Diethylether ausgefällt, wobei ein weißes Polymer erhalten wurde (0,36 g, 34 % d. Th.).
Molekulargewicht Mn (mittels GPC und NMR) ≈ 10.000; n ≈ 40
¹H-NMR (400 MHz, CDCl₃, ppm): 6,12 (s, 2H), 5,59 (s, 2H), 5,342 (s, 2H), 4,26 (t, J_{HH}=6,6 Hz, 80 H), 4,15 (t, 4H, J_{HH}=6,4 Hz), 4,08 (t, J_{HH}=6,6 Hz, 80 H), 3,65 (t, J_{HH}=6,4 Hz, 4H), 3,15 (t, J_{HH}=6,6 Hz, 80H), 2,67 (t, J_{HH}=6,6 Hz, 80H), 1,95 (s, 3H) 1,78-1,72 (m, 80H), 1,68-1,60 (m, 80H), 1,45-1,36 (m, 160H).

In den nachfolgenden Beispielen für die erfindungsgemäße Verwendung der Verbindungen der Formel (I) als Photoinitiatoren wurden jeweils Reaktionsgemische hergestellt, die den jeweiligen Photoinitiator, das angegebene flüssige Monomer und gegebenenfalls einen angegebenen Coinitiator enthielten und unter Stickstoffatmosphäre durch Bestrahlung mit einer OmniCure^{®} S2000-Quecksilberdampflampe mit einem Wellenlängenfilter von 320-500 nm und einer Intensität des UV-Lichts von 1 W/cm² jeweils 10 min lang gehärtet und der Verlauf der Reaktionen mittels Photo-DSC der Type NETSCH DSC 204 F1 Phönix verfolgt.

Alle Messungen wurden zumindest zweifach durchgeführt, wobei in den jeweiligen Tabellen die gemittelten Werte für den jeweiligen Initiator angegeben sind.

Darin gibt R_{P} die Polymerisationsrate an und ist somit ein Maß für die Reaktivität eines Systems. Ein hoher Wert bedeutet, dass sehr viele Monomergruppen gleichzeitig umgesetzt werden und dass die Aushärtung generell kürzer dauert. tₘₐₓ gibt die Zeit bis zum Erreichen des Maximums der Wärmeentwicklung (in s) an und ist somit ein Maß dafür, wie schnell der Gelpunkt und damit eine gewisse Anfangsfestigkeit erreicht sind. Dabei sind kurze Zeiten erstrebenswert. t_{95%} ist die Zeit (in s), nach der 95 % der gesamten Reaktionswärme freigesetzt waren, und ist also ein Maß für die Reaktionsgeschwindigkeit, wobei wiederum niedrige Werte vorteilhaft sind. Und DBC ist der Doppelbindungsumsatz der aus der bei der Polymerisation freigesetzte Reaktionswärme (in J pro g) für die jeweilige Formulierung berechnet wurde. Beim Reaktionsumsatz sollten natürlich möglichst hohe Werte erzielt werden.

Als Monomere wurden in allen Versuchen Acrylate und Methacrylate verwendet, die typischerweise in der Beschichtungstechnik zum Einsatz kommen.

Für alle Formulierungen wurde der jeweilige Photinitiator äquimolar auf 1 Gew.-% Ethylpyruvat (1), das den Initiator mit dem niedrigsten Molekulargewicht darstellt, bezogen und entsprechend eingewogen, und die Coinitiatoren wurden, sofern enthalten, äquimolar zum jeweiligen Initiator eingewogen. Danach wurden je 12 ± 0,5 mg der Reaktionsgemische in DSC-Schälchen aus Aluminium eingewogen und die Schälchen mit Deckgläsern bedeckt.

### Beispiele 1 bis 5, Vergleichsbeispiel 1 - Initiatoren vom Typ II

In dieser Versuchsgruppe wurden Wasserstoffabstraktoren der Beispiele 1 (nicht erfindungsgemäß) bis 4 (B1 bis B4) und eines Vergleichsbeispiels (V1) in Kombination mit einem Coinitiator als Wasserstoffdonor getestet.

### Initiatoren:

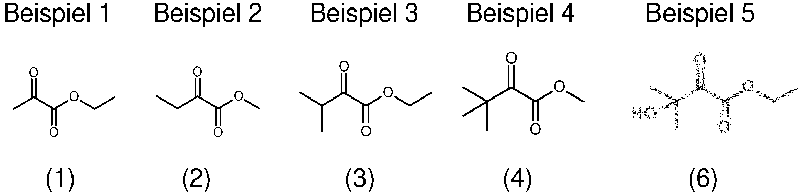

### Vergleichsbeispiel 1

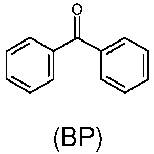

### Monomer.

### Hexandioldiacrylat (HDDA)

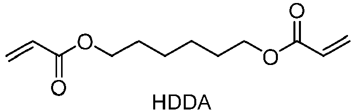

### Coinitiator.

### 4-Dimethylaminobenzoesäureethylester (DMAB)

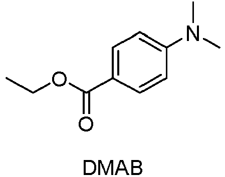

### Ergebnisse:

**Tabelle 1**

| | R_{P} [mmol.l⁻¹.s⁻¹] | tₘₐₓ [s] | t_{95%} [s] | DBC [%] |
|---|---|---|---|---|
| V1 | 96 | 10,1 | 85,5 | 68,9 |
| B1 | 230 | 7,7 | 41,7 | 66,5 |
| B2 | 204 | 8,5 | 40,5 | 60,9 |
| B3 | 241 | 7,5 | 36,6 | 65,8 |
| B4 | 170 | 10,6 | 42,5 | 59,1 |
| B5 | 218 | 6,0 | 73,0 | 63,1 |

Aus Tabelle 1 ist ersichtlich, dass die neuen α-Ketoester gemäß vorliegender Erfindung überraschend hohe Polymerisationsraten im Vergleich zur industriellen Referenz Benzophenon (BP) erreichen. Dies geht vor allem daraus hervor, dass die Zeit bis zum Erreichen von 95 % des Gesamtumsatzes t_{95%} deutlich geringer ist. Der Endumsatz (DBC) bleibt dabei auf einem vergleichbaren Niveau mit dem industriellen System BP/DMAB. Vor allem die Verbindungen der Beispiele 1 (nicht erfindungsgemäß) und 3 zeichnen sich durch eine hohe Reaktivität und einen besonders raschen Umsatz aus, wobei insbesondere das Methylethyloxobutanoat (3) aus Beispiel 3 hervorsticht.

### Beispiele 6 und 7, Vergleichsbeispiel 2 - Initiatoren vom Typ II

Zwei der obigen Versuche wurden unter Verwendung derselben beiden Initiatoren und erneut von BP als Vergleichsbeispiel wiederholt, wobei allerdings anstelle des aromatischen Amins (DMAB) ein nichtaromatischer Coinitiator (MDEA) eingesetzt wurde.

### Initiatoren:

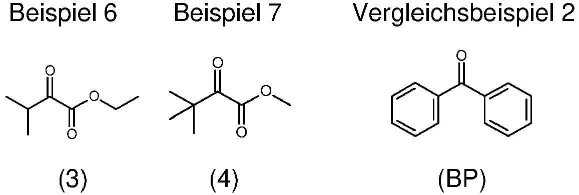

### Monomer.

### Hexandioldiacrylat (HDDA)

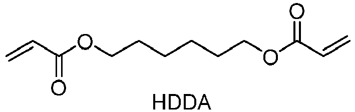

### Coinitiator.

### Methyldiethanolamin (MDEA)

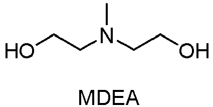

### Ergebnisse:

**Tabelle 2**

| | R_{P} [mmol.l⁻¹.s⁻¹] | tₘₐₓ [s] | t_{95%} [s] | DBC [%] |
|---|---|---|---|---|
| V2 | 109 | 10,8 | 77,0 | 69,3 |
| B6 | 228 | 6,7 | 56,5 | 65,2 |
| B7 | 234 | 7,7 | 41,0 | 64,6 |

Auch mit dem nichtaromatischen Coinitiator konnten mit den Verbindungen (3) und (4) gute Ergebnisse erzielt werden. Die beiden Ketoester zeigten deutlich höhere Reaktivität und eine niedrigere t_{95%} als das Referenzsystem.

### Beispiele 8 und 9, Vergleichsbeispiel 3 - Initiatoren vom Typ II

Die Verbindungen (1) (nicht erfindungsgemäß) und (4) wurden im Vergleich zum kommerziellen Initiator Ethylphenylglyoxylat (PGO) getestet.

### Initiatoren:

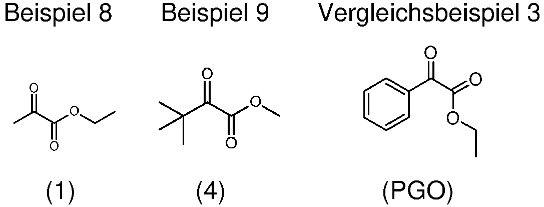

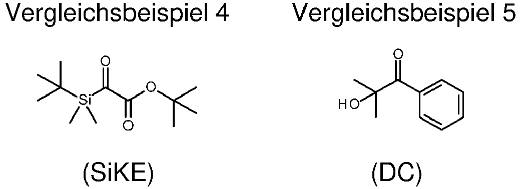

### Monomer.

### Hexandioldiacrylat (HDDA)

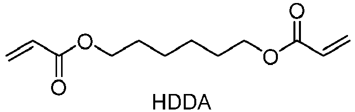

### Ergebnisse:

**Tabelle 3**

| | R_{P} [mmol.l⁻¹.s⁻¹] | tₘₐₓ [s] | t_{95%} [s] | DBC [%] |
|---|---|---|---|---|
| V3 | 204 | 14,1 | 63,0 | 75,1 |
| B8 | 210 | 8,5 | 56,0 | 69,3 |
| B9 | 214 | 8,3 | 53,5 | 64,6 |

Hier zeigte sich erstaunlicherweise, dass die neuen Verbindungen (1) (Beispiel 8; nicht erfindungsgemäß) und (4) (Beispiel 9) dem bekannten Phenylglyoxylat-Initiator (PGO) (Vergleichsbeispiel 3) in der Polymerisationsgeschwindigkeit deutlich überlegen sind. So wird die maximale Polymerisationsrate in nahezu der Hälfte der Zeit erreicht. Auch der Endumsatz wurde mit den neuen α-Ketoestern um bis zu 10 Sekunden schneller erreicht.

### Beispiel 10 - Initiator vom Typ II

Jene Verbindung, die in den bisherigen Versuchen am besten abgeschnitten hatte, Verbindung (3), wurde mit einem Thiol als Coinitiator getestet, und die dabei erzielten Ergebnisse wurden mit jenen aus Beispiel 3 mit DMAB als Coinitiator verglichen.

### Initiator.

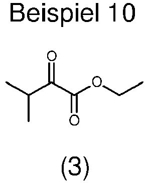

### Monomer.

### Hexandioldiacrylat (HDDA)

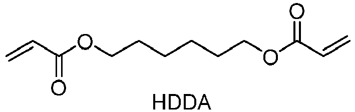

### Coinitiator.

### Pentaerythrit-tetrakis(3-mercaptopropionat) (Thiol)

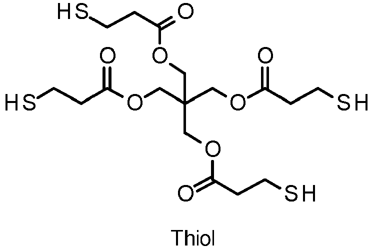

### Ergebnisse:

**Tabelle 4**

| | R_{P} [mmol.l⁻¹.s⁻¹] | tₘₐₓ [s] | t_{95%} [s] | DBC [%] |
|---|---|---|---|---|
| B3 | 241,0 | 7,5 | 36,6 | 65,8 |
| B10 | 233,5 | 8,0 | 45,0 | 68,0 |

Man erkennt, dass sich auch das Thiol sehr gut als Coinitiator für den α-Ketoester-Initiator vom Typ II eignet.

### Beispiel 11, Vergleichsbeispiel 4 - Initiatoren vom Typ I

In diesem Versuch wurde der bromierte Ketoester (5) aus Synthesebeispiel 1 mit einem ebenfalls bromhältigen, bekannten Initiator, "Bromdarocur" (BD), verglichen. Da es sich um Typ-I-Initiatoren handelt, erfolgte die Polymerisation ohne Coinitiator.

### Initiatoren:

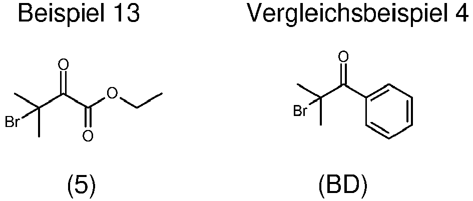

### Monomer.

### Hexandioldiacrylat (HDDA)

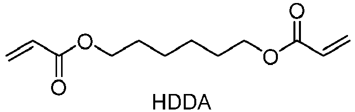

### Ergebnisse:

**Tabelle 5**

| | R_{P} [mmol.l⁻¹.s⁻¹] | tₘₐₓ [s] | t_{95%} [s] | DBC [%] |
|---|---|---|---|---|
| B11 | 239 | 5,9 | 40,0 | 62,1 |
| V4 | 146 | 7,3 | 62,5 | 57,6 |

Die erfindungsgemäße Verwendung des neuen α-Ketoesters (5) lieferte klar bessere Ergebnisse als der gängige bromhältige Typ-I-Initiator nach dem Stand der Technik, wobei die neue Verbindung (5) dem Letzteren in Bezug auf die Reaktivität überraschenderweise sogar deutlich überlegen ist.

### Beispiele 12 bis 15, Vergleichsbeispiel 5 - Initiatoren vom Typ II

In dieser Versuchsgruppe wurden die Experimente der Beispiele 1 bis 4 und des Vergleichsbeispiels 1 wiederholt, wobei jedoch anstelle des Diacrylats eine Dimethacrylatmischung aus der Dentaltechnik (DDM) als Monomer eingesetzt wurde.

### Initiatoren:

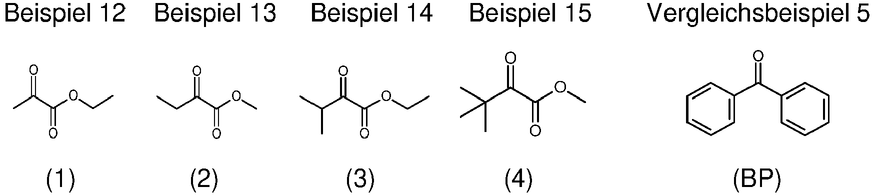

### Monomere:

### Dentale Dimethacrylatmischung (DDM)

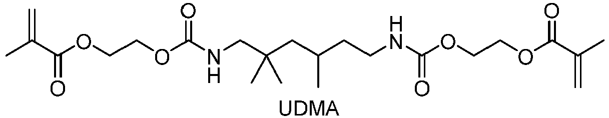

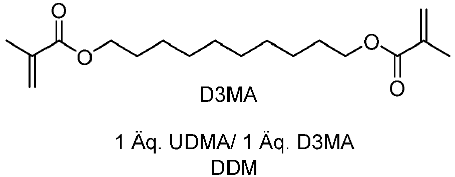

### Coinitiator.

### 4-Dimethylaminobenzoesäureethylester (DMAB)

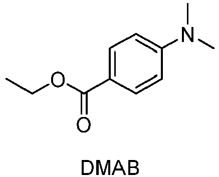

### Ergebnisse:

**Tabelle 7**

| | R_{P} [mmol.l⁻¹.s⁻¹] | tₘₐₓ [s] | t_{95%} [s] | DBC [%] |
|---|---|---|---|---|
| V5 | 21,4 | 18,5 | 146,5 | 50 |
| B12 | 74,5 | 11,9 | 83,0 | 57 |
| B13 | 71,2 | 12,2 | 85,5 | 57 |
| B14 | 81,5 | 11,0 | 58,5 | 57 |
| B15 | 55,9 | 14,9 | 69,5 | 51 |

Man erkennt, dass auch das Methacrylat-Monomer unter Verwendung von α-Ketoestern als Initiatoren besser härtbar ist als mit Benzophenon. Allerdings beträgt die Reaktivität der Ketoester (1) bis (4) hier überraschenderweise ein Vielfaches - nämlich zumindest das 2,5-fache - jener von Benzophenon, und die Zeit bis zum Erreichen des Peakmaximums ist um einiges kürzer als im Vergleichsbeispiel. In den Beispielen 12 (nicht erfindungsgemäß) und 13 bis 15 war mit dem Methacrylat-Monomer nun aber sogar der Doppelbindungsumsatz deutlich höher als mit dem Referenzinitiator, wobei dieser noch dazu 2- bis 3-mal so schnell erreicht war, was die Überlegenheit der vorliegende Erfindung gegenüber dem Stand der Technik noch weiter unterstreicht.

### Beispiel 16, Vergleichsbeispiel 6 - Initiatoren vom Typ II

In diesem Beispiel (nicht erfindungsgemäß) wurden ein Ketoester, Verbindung (1), mittels Photo-DSC-Messungen direkt mit Benzophenon (BP) als bekanntem Typ-II- Initiator bei der Härtung eines bekannten difunktionellen Vinylesters, nämlich Divinyladipat (DVA), als Monomer verglichen. Als Coinitiator wurde erneut p-Dimethylaminoethylbenzoat (DMAB) eingesetzt.

### Initiatoren:

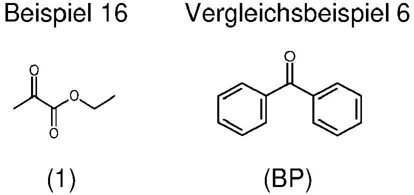

### Monomer.

### Divinyladipat (DVA)

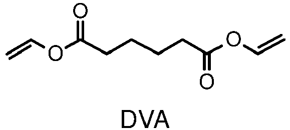

### Coinitiator.

### 4-Dimethylaminobenzoesäureethylester (DMAB)

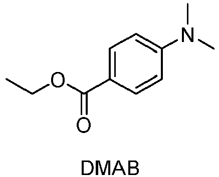

### Ergebnisse:

**Tabelle 8**

| | DSC [mW.mg⁻¹] | tₘₐₓ [s] | Fläche [J.g⁻¹] |
|---|---|---|---|
| B16 | 1,7 | 194 | 466 |
| V6 | 4,37 | 55 | 642 |

Man erkennt, dass bei Verwendung des im Vergleich zu (Meth)acrylaten sehr unreaktiven Vinylester-Monomers DVA der Ketoester (1) deutlich schlechter abschneidet als das Benzophenon im Vergleichsbeispiel. Dennoch zeigt dieser Vergleich, dass prinzipiell auch biokompatible Monomere wie DVA mit lebensmittelechten α-Ketoestern gehärtet werden können. Da Benzophenon und andere kommerzielle Initiatoren gesundheitlich bedenklich sein können, macht der Vorteil der Einsetzbarkeit die geringere Reaktivität bei Vinylestern wett.

### Beispiele 17 und 18, Vergleichsbeispiel 7 - Initiatoren vom Typ II in Hydrogelen

Die teilweise wasserlöslichen Ketoester konnten in einer Hydrogelformulierung mit PEG700DA (Polyethylenglykoldiacrylat mit Mn 700) und 50 Gew.-% Wasser überraschend gut eingesetzt werden um Hydrogele zu polymerisieren. Als kommerzieller Referenz-Initiator wurde BPQ mit MDEA als Coinitiator gewählt. Die Ketoester Ethylpyruvat (1) (nicht erfindungsgemäß) und Dimethylfurandion (8) können als neue Initiatoren ohne Coinitiator eingesetzt werden. Verbindung (8) kann aufgrund des langwelligen Absorptionsmaximums (λₘₐₓ = 378 nm statt 330 nm bei Verbindung (1)) auch bei für Zellen unschädlichem sichtbarem Licht eingesetzt werden.

### Initiatoren:

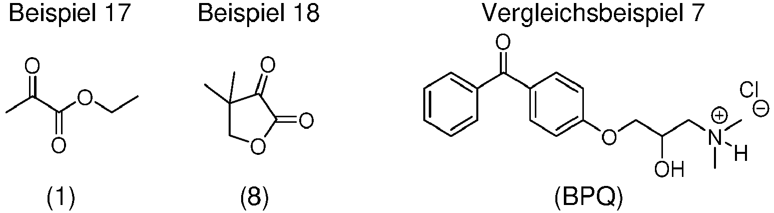

### Monomer:

### Polyethylenglykol(700)-diacrylat

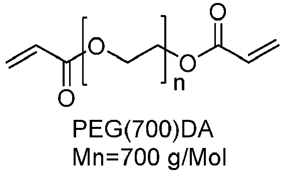

### Coinitiator:

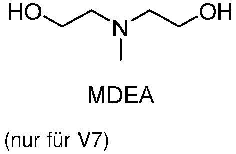

### Ergebnisse:

**Tabelle 9**

| | R_{P} [mmol.l⁻¹.s⁻¹] | tₘₐₓ [s] | t_{95%} [s] | DBC [%] |
|---|---|---|---|---|
| V7 | 30 | 8,8 | 87 | 50 |
| B17* | 33 | 8,5 | 83 | 48 |
| B18* | 26 | 10,3 | 78 | 56 |
| *ohne Coinitiator | | | | |

Wie aus in Tabelle 9 zu erkennen, zeigten sich die biokompatiblen Initiatoren als überraschend reaktiv und vergleichbar zum kommerziellen Initiator. Dazu ist fetszustellen, dass bei der kommerziellen Verbindung BPQ in einer Studie mit der Maus-Fibroblasten-Zellenlinie L929 bereits ab einer Konzentration von 8 mmol/I deutlich toxische Effekte auftraten, während sich bei den Verbindungen (1) (nicht erfindungsgemäß) und (8) auch bei der doppelten Konzentration keinerlei Zytotoxizität zeigte. Das kann ein zusätzlicher Vorteil für biologische Anwendungen sein, bei denen üblicherweise Hydrogele zum Einsatz kommen.

### Beispiel 19 und 20, Vergleichsbeispiele 8 und 9 - Initiatoren vom Typ II

Höchst überraschenderweise war die biokompatible Verbindung (8) aus Beispiel 18 in wässrigen und nichtwässrigen Monomersystemen gleichsam löslich. Deshalb wurde die Reaktivität auch in Acrylaten (Beispiel 19) und Methacyylaten (Beispiel 20) getestet, wobei in letzterem Fall aufgrund der geringeren Reaktivität der Methacrylate zusätzlich DMAB als Coinitiator eingesetzt wurde. Das neue System wurde mit dem industriellen Standardinitiator Benzophenon (BP) in Acrylaten (Vergleichsbeispiel 8) und Methacrylaten (Vergleichsbeispiel 9) verglichen.

### Initiatoren:

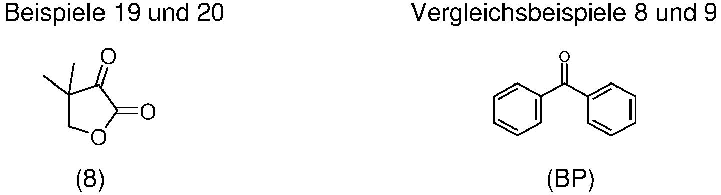

### Monomere:

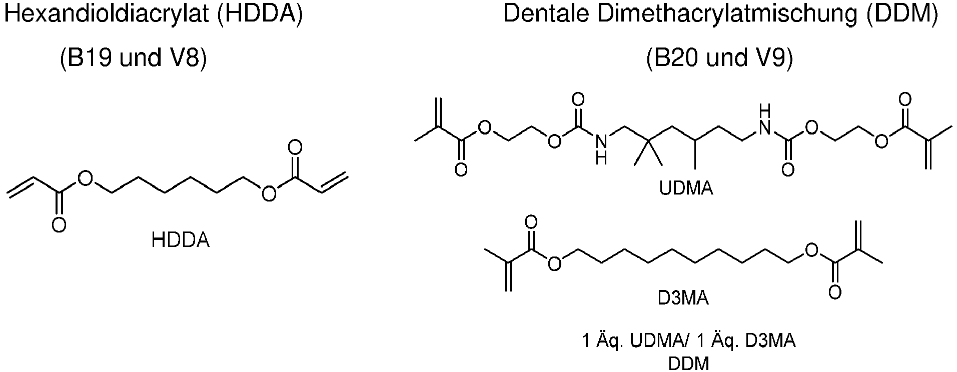

### Coinitiator.

### (B20, V8 und V9)

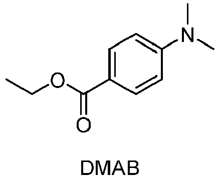

### Ergebnisse:

**Tabelle 10**

| | R_{P} [mmol.l⁻¹.s⁻¹] | tₘₐₓ [s] | t_{95%} [s] | DBC [%] |
|---|---|---|---|---|
| V8 | 93 | 11,1 | 69 | 69 |
| V9 | 21 | 18,2 | 143 | 49 |
| B19 | 189 | 11,6 | 58 | 66 |
| B20 | 52 | 18,2 | 90 | 54 |

Wie aus Tabelle 10 zu erkennen, zeigte die erfindungsgemäße Verbindung (8) erstaunlicherweise auch ohne Coinitiator in HDDA (Beispiel 19) eine mehr als doppelt so hohe Reaktivität wie die Referenz BP mit Coinitiator (Vergleichsbeispiel 8). Auch wird der ähnlich hohe Endumsatz rascher erreicht. In Methacrylaten wird mit DMAB als Coinitiator (Beispiel 20) von Verbindung (8) nicht nur eine mehr als doppelt so hohe Polymerisationsrate erreicht, sondern zugleich unerwarteterweise auch ein deutlich höherer Endumsatz wesentlich rascher erreicht als im Referenzbeispiel (Vergleichsbeispiel 9).

### Beispiel 21 und 22, Vergleichsbeispiel 10 - Initiatoren vom Typ II

Um polymerisierbare Initiatoren herzustellen, wurde Ketoglutarsäure mit einfachen Alkoholen (Beispiel 21) und Hydroxyethylmethacrylat (HEMA) (Beispiel 22) verestert und die Reaktivität im Diacrylat HDDA getestet. Die Reaktivität wurde wie zuvor mit einem Benzophenon-Amin-System verglichen (Vergleichsbeispiel 10).

### Initiatoren:

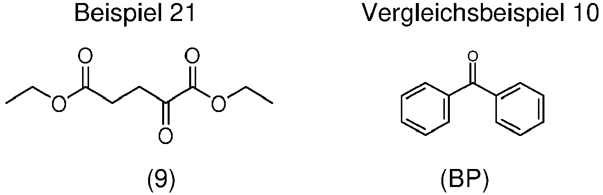

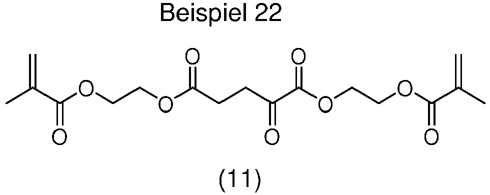

### Monomer.

### Hexandioldiacrylat (HDDA)

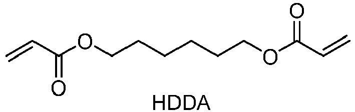

### Coinitiator.

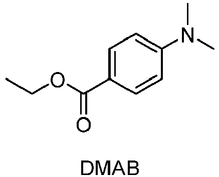

### Ergebnisse:

**Tabelle 11**

| | R_{P} [mmol.l⁻¹.s⁻¹] | tₘₐₓ [s] | t_{95%} [s] | DBC [%] |
|---|---|---|---|---|
| V10 | 93 | 11,1 | 69 | 69 |
| B21 | 201 | 9,2 | 58 | 67 |
| B22 | 203 | 11,6 | 53 | 64 |

Überraschenderweise zeigten auch diese α-Ketoester eine mindestens doppelt so hohe Polymerisationsrate wie das BP-Amin-Referenzsystem in Vergleichsbeispiel 10. Auch ein hoher Endumsatz wird schneller erreicht. Zusätzlich sorgen die polymerisierbaren Gruppen von Verbindung (11) dafür, dass restlicher Initiator nach der Polymerisation nicht mehr aus dem Polymer diffundieren kann, was für Anwendungen im Medizin- und Lebensmittelbereich von besonderer Bedeutung ist.

### Beispiel 23 und 24 und Vergleichsbeispiel 11 - Initiatoren vom Typ II

Auch in Methacrylaten lassen sich α-Ketoglutarsäureester einsetzen. Deshalb wurden die Substanzen (9) und (11) im Vergleich zu Benzophenon (BP) mit DMAB als Co-initiator in der Dentalformulierung DDM getestet.

### Initiatoren:

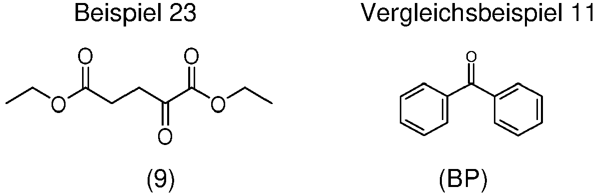

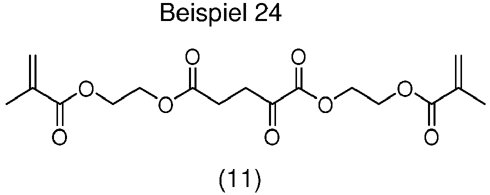

### Monomere:

### Dentale Dimethacrylatmischung (DDM)

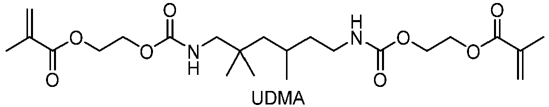

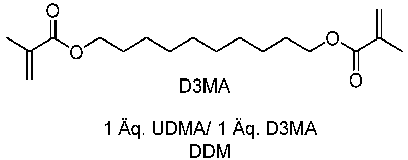

### Coinitiator.

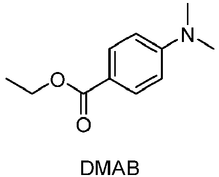

### Ergebnisse:

**Tabelle 12**

| | R_{P} [mmol*l⁻¹*s⁻¹] | tₘₐₓ [s] | t_{95%} [s] | DBC [%] |
|---|---|---|---|---|
| V11 | 21 | 18,2 | 143 | 49 |
| B23 | 66 | 14,3 | 85 | 53 |
| B24 | 57 | 14,5 | 93 | 52 |

Erstaunlicherweise lässt sich mit den neuen α-Ketoglutarsäureestern auch in unreaktiven Methacrylaten im Vergleich zum Referenzsystem eine 3-mal so hohe Polymerisationsgeschwindigkeit unter Verwendung von DMAB als Coinitiator feststellen, wie dies aus Tabelle 12 hervorgeht. Die Polymerisation läuft deutlich schneller ab als bei der Referenzsubstanz BP und führt auch rascher zu höheren Endumsätzen.

### Beispiel 25, Vergleichsbeispiel 12 - Polymere Initiatoren vom Typ II

Da es sich bei α-Ketoglutarsäure um eine Dicarbonsäure handelt, konnten auch Polyester aus Hexandiol und α-Ketoglutarsäure hergestellt werden. Polymere Initiatoren haben den Vorteil, nicht aus dem resultierenden Polymer zu migrieren und finden daher besonders im Lebensmittel- und Medizinbereich Anwendung. Zusätzlich lassen sich Polyester auf viele Arten modifizieren und so in puncto Löslichkeit, Funktionalität und Abbaubarkeit an die Anwendung anpassen.

### Initiatoren:

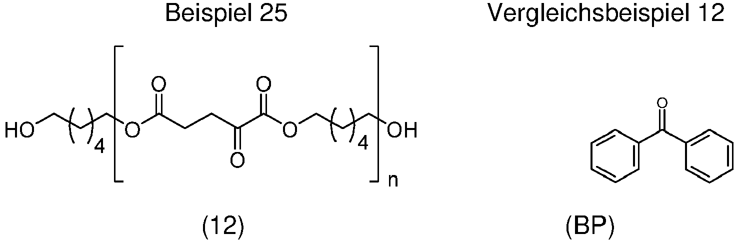

### Monomer.

### Hexandioldiacrylat (HDDA)

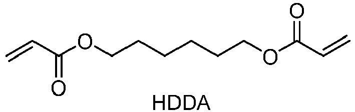

### Coinitiator.

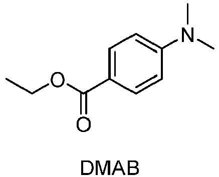

### Ergebnisse:

**Tablle 13**

| | R_{P} [mmol.l⁻¹.s⁻¹] | tₘₐₓ [s] | t_{95%} [s] | DBC [%] |
|---|---|---|---|---|
| V12 | 93 | 11,1 | 69 | 69 |
| B25 | 144 | 9,2 | 71 | 64 |

Wie Tabelle 13 veranschaulicht, zeigt der Polyester (12) in Beispiel 25 unerwartet hohe Reaktivität im Vergleich zu Benzophenon (BP) in Vergleichsbeispiel 12. Trotz des hohen Molekulargewichts und der damit verbundenen langsamen Diffusion ist die Polymerisationsrate um mehr als ein Drittel erhöht. α-Ketoglutarsäure-Polyester sind für ihre Biokompatibilität und ihre biologische Abbaubarkeit bekannt. Der Einsatz dieser Substanzklasse als Photoinitiator ist jedoch völlig neu, und die hohe Polymerisationsrate war sehr überraschend.

### Beispiel 26 - Initiator vom Typ II, Härtungsversuch

Um die Reaktivität von Typ-II-Initiatoren zu erhöhen, werden Amine als Coinitiatoren eingesetzt. Zur Vermeidung eines Zweikomponentensystems kann der Coinitiator auch kovalent an den Initiator gebunden sein. Verbindung (10) stellt einen solchen Initiator dar, der in diesem Beispiel mit Hexandioldiacrylat (HDDA) als Monomer getestet wurde.

### Initiator.

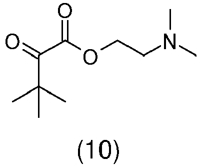

### Monomer.

### Hexandioldiacrylat (HDDA)

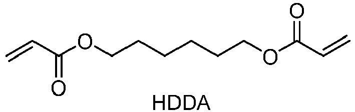

Ein Gramm eines Gemischs aus HDDA und 1 Gew.-% an Verbindung (10) wurde in einer Silikonform in einem Intelli-Ray 400 W UV-Ofen bei 100 % Leistung bestrahlt. Nach wenigen Sekunden wurde ein klarer, harter Probestab erhalten, was die Eignung der vorliegenden Erfindung für die Herstellung von Formkörpern belegt.

### Beispiel 27 - Polymerer Initiator vom Typ II, Härtungsversuch

Unerwünschte Migration von nichtumgesetzten Initiatormolekülen oder deren Zersetzungsprodukten bei Bestrahlung stellen besonders im Lebensmittel- und Medizinbereich ein großes Problem dar. Deshalb sind makromolekulare Initiatoren und im Besonderen makromolekulare Initiatoren mit polymerisierbaren Endgruppen von gro-ßem Interesse. Durch das hohe Molekulargewicht und die reaktiven Endgruppen kann der Initiator nicht mehr aus dem resultierenden Polymer diffundieren. Zu diesem Zweck wurde Verbindung (13) mit Hexandioldiacrylat (HDDA) als Monomer untersucht.

### Initiator.

### Beispiel 27

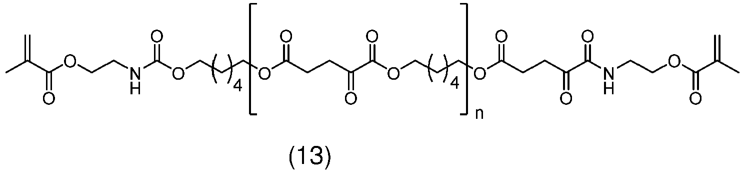

### Monomer.

### Hexandioldiacrylat (HDDA)

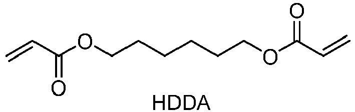

Ein Gramm eines Gemischs aus HDDA und 1 Gew.-% an Verbindung (13) wurde in einer Silikonform in einem Intelli-Ray 400 W UV-Ofen bei 100 % Leistung bestrahlt. Nach wenigen Sekunden wurde ein klarer, harter Probestab erhalten, was auch die Eignung des polymeren Initiators gemäß vorliegender Erfindung bei der Herstellung von Formkörpern belegt.

Die obigen Beispiele belegen, dass die erfindungsgemäße Verwendung von α-Ketoestern je nach Reaktionsbedingungen eine Reihe von Vorteilen gegenüber bekannten Initiatoren bietet, so dass die vorliegende Erfindung eine wertvolle Bereicherung des Standes der Technik darstellt.

## Patentansprüche

1. Verwendung von Verbindungen der nachstehenden Formel (I) als Photoinitiator oder Photosensitizer in einer photopolymerisierbaren Zusammensetzung: 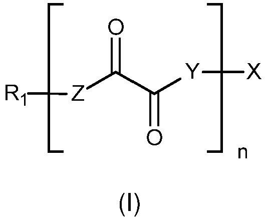 worin
R₁ für einen einwertigen, linearen, verzweigten oder zyklischen aliphatischen Kohlenwasserstoffrest mit 1 bis 20 Kohlenstoffatomen steht, der gegebenenfalls durch ein oder mehrere Heteroatome, ausgewählt aus -O- und -NR₂- unterbrochen ist und der gegebenenfalls mit einem oder mehreren Substituenten, ausgewählt aus -Cl, -Br, -OH, =O, -NH-CO-OR₂, -NH-CO-R₂ und radikalisch oder ionisch polymerisierbaren Gruppierungen substituiert ist, wobei die Reste R₂ jeweils unabhängig aus -H und C₁₋₆-Alkyl ausgewählt sind;
n ≥ 1 ist, wobei
i) wenn n = 1 ist, Z und Y fehlen und X für -OR₃ steht; und
ii) wenn n > 1 ist, Z für -OR₄- steht, Y für -OR₅- steht und X für -H oder -OH steht; worin
R₃ für -H oder eine der Optionen für R₁ steht; und
R₄ und R₅ jeweils unabhängig für einen zweiwertigen Kohlenwasserstoffrest stehen, für den ansonsten dieselben Optionen wie für R₁ gelten;
wobei die polymerisierbaren Gruppierungen als optionale Substituenten von R₁ aus polymerisierbaren Doppel- und Dreifachbindungen sowie aus der Ringöffnungspolymerisation zugänglichen Lactam-, Lacton- und Epoxid-Gruppierungen ausgewählt sind;
und wobei beliebige zwei der Reste R₁ bis R₅ gegebenenfalls miteinander verbunden sind, um einen Ring oder ein Dimer zu bilden;
mit der Maßgabe, dass die Verbindung der Formel (I) nicht 2-Oxopropansäureethylester (1) ist.

2. Verwendung nach Anspruch 1, **dadurch gekennzeichnet, dass** in Formel (I):
a) R₁ für einen einen linearen, verzweigten oder zyklischen aliphatischen Kohlenwasserstoffrest mit 1 bis 20 Kohlenstoffatomen steht, in dem gegebenenfalls ein oder mehrere Kohlenstoffatome durch Sauerstoffatome ersetzt sind und der gegebenenfalls mit einem oder mehreren Substituenten, ausgewählt aus -Cl, -Br, -OH und -SH substituiert ist; und/oder
b1) n = 1 ist und X für -OR₃ steht, wobei R₃ jeweils unabhängig für -H oder eine der Optionen für R₁ steht; oder
b2) n > 1 und aus dem Zahlenbereich von 2 bis 100 oder von 2 bis 50 oder von 2 bis 20 ausgewählt ist und X für -OH steht.

3. Verwendung nach Anspruch 1 oder 2, **dadurch gekennzeichnet, dass** die Verbindung der Formel (I) als Photoinitiator vom Typ I oder Typ II eingesetzt wird.

4. Verwendung nach einem der Ansprüche 1 bis 3, **dadurch gekennzeichnet, dass** die Verbindung der Formel (I) in den photopolymerisierbaren Zusammensetzungen als Photoinitiator in Kombination mit einem oder mehreren Coinitiatoren eingesetzt wird.

5. Verwendung nach Anspruch 4, **dadurch gekennzeichnet, dass** als Coinitiator eine oder mehrere Verbindungen eingesetzt werden, die aus ein- oder mehrwertigen Alkoholen (-OH), Thiolen (-SH), Aminen (-NR-), Silanen (=SiH-), Germanen (=GeH-), Phosphinen (-PRR'R"-), Ethern (>CH-O-CH<), lodonium- (-I⁺-) und Sulfonium- (=S⁺-) Salzen sowie Derivaten davon basierenden Verbindungen ausgewählt sind.

6. Verwendung nach Anspruch 5, **dadurch gekennzeichnet, dass** als Coinitiator eine oder mehrere Verbindungen, ausgewählt aus Zuckern, Glycerin, Thiolen, Polyethylenglykol und Polypropylenglykol eingesetzt werden, wobei vorzugsweise Glycerin als Coinitiator eingesetzt wird.

7. Verwendung nach einem der Ansprüche 1 bis 8, **dadurch gekennzeichnet, dass** die Verbindung der Formel (I) in einer Menge von 0,1 bis 10 Gewichtsteilen, vorzugsweise 0,5 bis 5 Gewichtsteilen, insbesondere 1 bis 2 Gewichtsteilen, pro 100 Gewichtsteile an polymerisierbaren Monomeren eingesetzt wird.

8. Verwendung nach einem der Ansprüche 1 bis 7, **dadurch gekennzeichnet, dass** die Verbindung der Formel (I) zur Härtung von Acrylat- oder Methacrylat-Monomeren, vorzugsweise von Methacrylat-Monomeren, eingesetzt wird.

9. Verwendung nach einem der Ansprüche 1 bis 8, **dadurch gekennzeichnet, dass** die Verbindung der Formel (I) als Initiator in Zusammensetzungen, die innerhalb des menschlichen Körpers gehärtet werden sollen, oder für härtbare Zusammensetzungen auf dem Lebensmittelsektor eingesetzt wird.

10. Photopolymerisierbare Zusammensetzung, **dadurch gekennzeichnet, dass** sie zumindest eine Verbindung der Formel (I), die nicht 2-Oxopropansäureethylester (1) ist, als Photoinitiator oder Photosensitizer, zumindest ein photopolymerisierbares Monomer und gegebenenfalls zumindest einen Coinitiator, wie in einem der Ansprüche 1 bis 8 definiert, sowie gegebenenfalls weitere Komponenten, wie z.B. Füllstoffe, Lösungsmittel, weitere Initiatoren oder Sensitizer, Weichmacher oder Fließhilfen, umfasst.

## Claims

1. A use of compounds of the following formula (I) as photoinitiators or photosensitizers in a photopolymerizable composition: 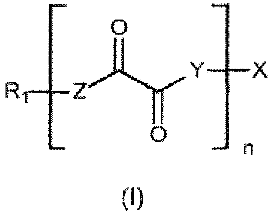 wherein
R₁ represents a monovalent, linear, branched or cyclic aliphatic hydrocarbon residue having 1 to 20 carbon atoms, which is optionally interrupted by one or more heteroatoms selected from -O- and -NR₂- and which is optionally substituted with one or more substitutes selected from -Cl, -Br, -OH, =O, -NH-CO-OR₂, -NH-CO-R₂ and free-radically or ionically polymerizable moieties, wherein the residues R₂ are each independently selected from -H and C₁₋₆ alkyl;
n is ≥ 1, wherein
i) if n = 1, Z and Y are not present and X represents -OR₃; and
ii) if n is > 1, Z represents -OR₄-, Y represents -OR₅-, and X represents -H or -OH; wherein
R₃ represents -H or one of the options mentioned for R₁; and
R₄ and R₅ each independently represent a bivalent hydrocarbon residue, for which otherwise the same options apply as for R₁;
wherein the polymerizable moieties as optional substituents of R₁ are selected from polymerizable double or triple bonds as well as from lactam, lactone, and epoxide moieties, which are subjectable to ring-opening polymerization;
and wherein any two of the residues R₁ to R₅ may be optionally linked to each other to form a ring or a dimer;
with the proviso that the compound of formula (I) is not 2-oxopropionic acid ethyl ester (1).

2. The use according to claim 1, **characterized in that** in formula (I):
a) R₁ represents a linear, branched or cyclic aliphatic hydrocarbon residue having 1 to 20 carbon atoms, in which optionally one or more carbon atoms are replaced by oxygen atoms and which is optionally substituted with one or more substituents selected from -Cl, -Br, -OH-, and -SH; and/or
b1) n = 1 and X represents -OR₃, wherein each R₃ independently represents -H or one of the options mentioned for R₁; or
b2) n is > 1 and is selected from the numerical range from 2 to 100 or from 2 to 50 or from 2 to 20, and X represents -OH.

3. The use according to claim 1 or 2, **characterized in that** the compound of formula (I) is used as a type I or type II photoinitiator.

4. The use according to any one of claims 1 to 3, **characterized in that** the compound of formula (I) is used in the photopolymerizable compositions as a photoinitiator in combination with one or more co-initiators.

5. The use according to claim 4, **characterized in that** one or more compounds are used as co-initiators, which compounds are selected from monohydric or polyhydric alcohols (-OH), thiols (-SH), amines (-NR-), silanes (=SiH-), germanes (=GeH-), phosphines (-PRR'R"-), ethers (>CH-O-CH<), iodonium (-I⁺-) and sulfonium (=S⁺-) salts as well as compounds based on derivatives thereof.

6. The use according to claim 5, **characterized in that** one or more compounds are used as co-initiators, which compounds are selected from sugars, glycerol, thiols, polyethylene glycol, and polypropylene glycol, with glycerol preferably being used as a co-initiator.

7. The use according to any one of the claims 1 to 6, **characterized in that** the compound of formula (I) is used in an amount of 0.1 to 10 parts by weight, preferably 0.5 to 5 parts by weight, most preferably 1 to 2 parts by weight, per 100 parts by weight of polymerizable monomers.

8. The use according to any one of the claims 1 to 7, **characterized in that** the compound of formula (I) is used for curing acrylate or methacrylate monomers, preferably methacrylate monomers.

9. The use according to any one of the claims 1 to 8, **characterized in that** the compound of formula (I) is used as an initiator in compositions to be cured inside the human body or for curable compositions in the food industry.

10. A photopolymerizable composition, **characterized in that** it comprises at least one compound of formula (I) other than 2-oxopropane acid ethyl ester (1) as a photoinitiator or photosensitizer, at least one photopolymerizable monomer, and optionally at least one co-initiator, as defined in any one of the claims 1 to 8, and optionally further components such as fillers, solvents, further initiators or sensitizers, plasticizers or flow promoters.

## Revendications

1. Usage de composés de formule (I) ci-dessous comme photoinitiateur ou photosensibilisateur dans une composition photopolymérisable: 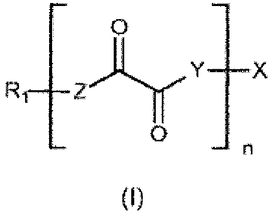 dans laquelle
R₁ représente un résidu hydrocarburé aliphatique monovalent, linéaire, ramifié ou cyclique, ayant 1 à 20 atomes de carbone, qui est éventuellement interrompu par un ou plusieurs hétéroatomes sélectionnés parmi -O- and -NR₂- et qui est éventuellement substitué par un ou plusieurs substituants sélectionnés parmi -Cl, -Br, -OH, =O, -NH-CO-OR₂, -NH-CO-R₂ et des groupements polymérisables par voie radicalaire ou ionique, les résidus R₂ étant chacun sélectionnés indépendamment parmi -H and alkyle C₁₋₆ ;
n ≥ 1, dans lequel
i) si n = 1, Z et Y sont absents et X représente -OR₃ ; et
ii) si n > 1, Z représente -OR₄-, Y représente -OR₅- et X représente -H ou -OH ; dans lequel
R₃ représente -H ou une des options pour R₁ ; et
R₄ et R₅ représentent chacun indépendamment un résidu hydrocarburé bivalent, pour lequel s'appliquent par ailleurs les mêmes options que pour R₁ ;
dans lequel les groupements polymérisables sont sélectionnées en tant que substituants optionnels de R₁ parmi des doubles ou triples liaisons polymérisables ainsi que des groupements de lactame, de lactone et d'époxyde ;
et dans lequel deux quelconques parmi les résidus R₁ à R₅ sont éventuellement liés entre eux pour former un cycle ou un dimère ;
à condition que le composé de formule (I) ne soit pas un ester éthylique d'acide 2-oxopropionique (1).

2. Usage selon la revendication 1, **caractérisé en ce que** dans la formule (I):
a) R₁ représente un résidu hydrocarburé aliphatique monovalent, linéaire, ramifié ou cyclique, ayant 1 à 20 atomes de carbone, dans lequel éventuellement un ou plusieurs atomes de carbone sont remplacés par des atomes d'oxygène et qui est éventuellement substitué par un ou plusieurs substituants sélectionnés parmi -Cl, -Br, -OH- et -SH ; et/ou
b1) n = 1 et X représente -OR₃, dans lequel chaque R₃ représente indépendamment -H ou une des options pour R₁ ; ou
b2) n > 1 et est sélectionné du domaine numérique de 2 à 100 ou de 2 à 50 ou de 2 à 20, et X représente -OH.

3. Usage selon l'une quelconque parmi les revendications 1 ou 2, **caractérisé en ce que** le composé de formule (I) est utilisé comme photoinitiateur de type I ou type II.

4. Usage selon l'une quelconque parmi les revendications 1 à 3, **caractérisé en ce que** le composé de formule (I) est utilisé dans les compositions photopolymérisables comme photoinitiateur en combinaison avec un ou plusieurs co-initiateurs.

5. Usage selon la revendication 4, **caractérisé en ce qu'**un ou plusieurs composés sont utilisés comme co-initiateurs, lesdits composés étant sélectionnés parmi les alcools monohydriques ou polyhydriques (-OH), les thiols (-SH), les amines (-NR-), les silanes (=SiH-), les germanes (=GeH-), les phosphines (-PRR'R"-), les éthers (>CH-O-CH<) les sels de iodonium (-I⁺-) et sulfonium (=S⁺-) et des composés à base de leurs dérivés.

6. Usage selon la revendication 5, **caractérisé en ce qu'**un ou plusieurs composés sont utilisés comme co-initiateurs, lesdits composés étant sélectionnés parmi les sucres, le glycérol, les thiols, le polyéthylène glycol et le polypropylène glycol, le co-initiateur utilisé étant de préférence le glycérol.

7. Usage selon l'une quelconque parmi les revendications 1 à 6, **caractérisé en ce que** le composé de formule (I) est utilisé en une quantité comprise entre 0,1 et 10 parties en poids, de préférence entre 0,5 et 5 parties en poids, notamment entre 1 et 2 parties en poids, par 100 parties en poids de monomères polymérisables.

8. Usage selon l'une quelconque parmi les revendications 1 à 7, **caractérisé en ce que** le composé de formule (I) est utilisé pour durcir des monomères d'acrylate ou de méthacrylate, de préférence des monomères de méthacrylate.

9. Usage selon l'une quelconque parmi les revendications 1 à 8, **caractérisé en ce que** le composé de formule (I) est utilisé comme initiateur dans des compositions destinées à être durcies dans le corps humain ou pour des compositions durcissables dans le domaine des produits alimentaires.

10. Composition photopolymérisable, **caractérisée en ce qu'**elle comprend au moins un composé de formule (I), qui n'est pas le l'ester éthylique d'acide 2-oxopropionique (1), comme photoinitiateur ou photosensibilisateur, au moins un monomère photopolymérisable, et éventuellement au moins un co-initiateur, comme définis dans une quelconque parmi les revendications 1 à 8, et éventuellement d'autres composants tel que des charges, des solvants, d'autres initiateurs ou sensibilisateurs, des plastifiants ou des fluidifiants.
